# EUROPEAN PATENT APPLICATION

(11) **EP 4 465 036 A1**
(43) Date of publication of application: **20.11.2024**
(21) Application number: 22920561.2
(22) Date of filing: 16.12.2022
(51) Int. Cl.: G01N 27/62, G01N 33/68

(54) **METHOD FOR ANALYZING NEUROGRANIN-RELATED PEPTIDE**

(30) Priority: 13.01.2022 JP 2022003946
(71) Applicant: SHIMADZU CORPORATION, Kyoto-shi, Kyoto 604-8511 (JP)
(72) Inventor: KANEKO, Naoki, Kyoto-shi, Kyoto 604-8511 (JP)
(74) Representative: Müller-Boré & Partner Patentanwälte PartG mbB
(86) International application number: PCT/JP2022/046405
(87) International publication number: WO 2023/136043

(57) **Abstract**

Provided is a method for analyzing a neurogranin-related peptide by matrix assisted laser desorption/ionization-mass spectrometry, in which a laser irradiation target contains the neurogranin-related peptide, a matrix, and a protein of 9 kDa or more, and a content of the protein per 1 pg of the matrix is 10 fmol or more and 600 finol or less.

## Description

### TECHNICAL FIELD

The present invention relates to a method for analyzing a neurogranin-related peptide.

### BACKGROUND ART

Alzheimer's disease is the leading cause of dementia, and its afflicted people have increased more and more in recent years, and its research has become even more important. In the onset of Alzheimer's disease, Aβ-related peptides such as amidroid β (AB) generated by cleavage of amyloid precursor protein (APP) are deeply involved. Then, it has been reported that a plurality of Aβ-related peptides in blood are detected by combining immunoprecipitation and mass spectrometry, and the detected specific Aβ-related peptide ratio is promising as a blood biomarker of amyloid accumulation in the brain (Non-Patent Documents 1 and 2, Patent Documents 1 to 3).

On the other hand, Alzheimer's disease requires various biomarkers in order to monitor its disease progression, and there is a demand for biomarkers for reflecting each process of tau accumulation and neurodegeneration in addition to amyloid accumulation. Among them, neurogranin is one of biomarkers of neurodegeneration, and is reported to increase in cerebrospinal fluid (CSF) of Alzheimer's disease patients (Non-Patent Document 3 and Non-Patent Document 4). In addition, it has also been reported that fragmentation of neurogranin is promoted and fragment peptides are generated in the brain of Alzheimer's disease patients (Non-Patent Document 5). Therefore, mass spectrometry of neurogranin or a fragment peptide thereof (neurogranin-related peptide) is expected as a means for confirming neurodegeneration.

By the way, it is necessary to collect cerebrospinal fluid, and it is not preferable to analyze the neurogranin-related peptide in the cerebrospinal fluid from the viewpoint of invasiveness. Therefore, it is possible to collect blood by a general test, and it is desired to analyze blood that is minimally invasive.

However, since the neurogranin-related peptide present in blood also contains very small amounts of peptides, the neurogranin-related peptide cannot be sufficiently detected by conventional mass spectrometry. For example, it has been reported that neurogranin or the like could be detected by a method combining immunoprecipitation and mass spectrometry (Non-Patent Document 6).

### PRIOR ART DOCUMENTS

### PATENT DOCUMENTS

Patent Document 1: WO 2015/178398
Patent Document 2: WO 2017/47529
Patent Document 3: JP-A-2017-20980

### NON-PATENT DOCUMENTS

Non-Patent Document 1: Kaneko N, Nakamura A, Washimi Y, Kato T, Sakurai T, Arahata Y, Bundo M, Takeda A, Niida S, Ito K, Toba K, Tanaka K, Yanagisawa K. : Novel plasma biomarker surrogating cerebral amyloid deposition. Proc Jpn Acad Ser B Phys Biol Sci. 2014;90(9):353-364.

Non-Patent Document 2: Nakamura A, Kaneko N, Villemagne VL, Kato T, Doecke J, Dore V, Fowler C, Li QX, Martins R, Rowe C, Tomita T, Matsuzaki K, Ishii K, Ishii K, Arahata Y, Iwamoto S, Ito K, Tanaka K, Masters CL, Yanagisawa K. : High performance plasma amyloid-β biomarkers for Alzheimer's disease. Nature. 2018;554(7691):249-254.

Non-Patent Document 3: Portelius E, Olsson B, Hoglund K, Cullen NC, Kvartsberg H, Andreasson U, Zetterberg H, Sandelius A, Shaw LM, Lee VMY, Irwin DJ, Grossman M, Weintraub D, Chen-Plotkin A, Wolk DA, McCluskey L, Elman L, McBride J, Toledo JB, Trojanowski JQ, Blennow K. : Cerebrospinal fluid neurogranin concentration in neurodegeneration: relation to clinical phenotypes and neuropathology. Acta Neuropathol. 2018;136(3):363-376.

Non-Patent Document 4: Thorsell A, Bjerke M, Gobom J, Brunhage E, Vanmechelen E, Andreasen N, Hansson O, Minthon L, Zetterberg H, Blennow K. : Neurogranin in cerebrospinal fluid as a marker of synaptic degeneration in Alzheimer's disease. Brain Res. 2010;1362:13-22.

Non-Patent Document 5: Kvartsberg H, Lashley T, Murray CE, Brinkmalm G, Cullen NC, Hoglund K, Zetterberg H, Blennow K, Portelius E. : The intact postsynaptic protein neurogranin is reduced in brain tissue from patients with familial and sporadic Alzheimer's disease. Acta Neuropathol. 2019:137(1):89-102.

Non-Patent Document 6: Kvartsberg H, Portelius E, Andreasson U, Brinkmalm G, Hellwig K, Lelental N, Kornhuber J, Hansson O, Minthon L, Spitzer P, Maler JM, Zetterberg H, Blennow K, Lewczuk P. : Characterization of the postsynaptic protein neurogranin in paired cerebrospinal fluid and plasma samples from Alzheimer's disease patients and healthy controls. Alzheimers Res Ther. 2015;7(1):40.

### SUMMARY OF THE INVENTION

### PROBLEMS TO BE SOLVED BY THE INVENTION

However, even when the analysis method of Non-Patent Document 6 is performed and when the analysis method of the Aβ-related peptide disclosed in Non-Patent Document 2 is diverted to the neurogranin-related peptide, the analysis sensitivity is insufficient and has not reached the level of practical use. Therefore, further improvement in sensitivity is required for analyzing neurogranin and the like in blood.

An object of the present invention is to detect a neurogranin-related peptide with high sensitivity.

### MEANS FOR SOLVING THE PROBLEMS

A first aspect of the present invention is a method for analyzing a neurogranin-related peptide by matrix assisted laser desorption/ionization-mass spectrometry, in which a laser irradiation target contains the neurogranin-related peptide, a matrix, and a protein of 9 kDa or more, and a content of the protein with respect to 1 pg of the matrix is 10 fmol or more and 600 fmol or less.

### EFFECTS OF THE INVENTION

According to the first aspect of the present invention, a neurogranin-related peptide can be analyzed with high sensitivity.

### BRIEF DESCRIPTION OF THE DRAWINGS

FIG. 1 is a graph of Example 1 illustrating a variation rate of detection sensitivity of MALDI-MS in a case where a laser irradiation target contains recombiant Ng as a protein. A vertical axis represents a signal/noise ratio (S/N) of a peak when a protein (recombinant Ng) is contained to a S/N of a mass spectrum peak when the protein is not contained, and a horizontal axis represents a content of the protein to 1 pg of the matrix.
FIG. 2 is a graph of Example 2 illustrating a variation rate of detection sensitivity of MALDI-MS in a case where a laser irradiation target contains Cytochrome C as a protein.
FIG. 3 is a graph of Example 3 illustrating a variation rate of detection sensitivity of MALDI-MS in a case where a laser irradiation target contains Bovine serum albumin as a protein.
FIG. 4 is a graph of Example 4 illustrating a variation rate of detection sensitivity of a Ng43-75 peptide when a protein-containing solution is added dropwise to a MALDI plate. The vertical axis represents a signal/noise ratio (S/N) of a peak when a protein is contained to a S/N of a mass spectrum peak when the protein is not contained, and the horizontal axis represents the type of protein.
FIG. 5 is a graph of Example 4 illustrating a variation rate of detection sensitivity of a Ng33-75 peptide when a protein-containing solution is added dropwise to a MALDI plate.

### MODE FOR CARRYING OUT THE INVENTION

### 1. First embodiment

An analysis method according to a first embodiment is a method for analyzing a neurogranin-related peptide in a sample, and the method sequentially includes a purification step and a detection step. Hereinafter, each step will be described in detail.

The "neurogranin-related peptide" (hereinafter, the peptide is abbreviated as "Ng-related peptide") includes neurogranin, translated/modified neurogranin, and fragment peptides thereof. Examples of the fragment peptide include Ng43-75 (SEQ ID NO: 1), Ng33-75 (SEQ ID NO: 2), and Ng50-78 (SEQ ID NO: 3). The mass (molecular weight) of the Ng-related peptide is generally less than 9 kDa, preferably 8 kDa or less, and is, for example, 1 kDa or more and preferably 2 kDa or more. In the first embodiment, in particular, an Ng-related peptide of less than 9 KDa can be suitably measured. This mass can be measured by, for example, a MALDI-TOF (Matrix Assisted Laser Desorption/Ionization-Time of Flight) type mass spectrometer or the like.

### 1-1. Purification step

The purification step is, for example, affinity purification, and specifically includes a first binding step (an example of a binding step), a first washing step (an example of a washing step), a first elution step, a neutralization step, a second binding step, a second washing step, and a second elution step (an example of an elution step) in this order.

### (First binding step)

In the first binding step, a sample is brought into contact with a first carrier in a binding liquid. For example, a binding solution, the sample, and the first carrier are mixed in an appropriate order. As a result, the Ng-related peptide in the sample is bound to the first carrier, and thus a first conjugate is obtained.

The sample is a sample containing a neurogranin-related peptide, and is generally a biological sample. Examples of the biological sample include body fluids such as blood, cerebrospinal fluid, urine, body secretion, saliva, and sputum, and examples thereof include feces. The blood includes whole blood, plasma, serum, and the like. The blood may be obtained by subjecting whole blood collected from an individual to treatment such as centrifugation and cryopreservation. The present analysis method preferably includes blood. Blood is less invasive than cerebral bone marrow fluid, and is a target sample for screening in a medical examination or the like, and is easily available.

The binding solution is preferably a neutral buffer containing a surfactant. Examples of the buffer include a Tris buffer, a phosphate buffer, a HEPES buffer, and an ammonium acetate buffer. The pH of the binding solution is, for example, pH 6.0 or more and preferably 6.5 or more, and is, for example, 8.5 or less and preferably 8.0 or less.

Examples of the surfactant contained in the binding solution include a neutral surfactant having a hydrophobic group having 7 or more and 15 or less carbon atoms (preferably, 9 or more and 11 or less). This makes it possible to suppress non-specific adsorption to the first conjugate and to reduce ionization interference in mass spectrometry. Examples of such surfactants include surfactants having maltose in a hydrophilic moiety, such as n-nonyl-β-D-maltoside, n-nonyl-β-D-thiomaltoside, n-decyl-β-D-maltoside, and n-undecyl-β-D-maltoside (UDM); surfactants having trehalose in a hydrophilic moiety, such as, for example, α-D-glucopyranosyl α-D-glucopyranoside monodecanoate (trehalose C10); and surfactants having glucose in a hydrophilic moiety, such as n-decyl-β-D-glucoside. These surfactants can be used singly or in combination of two or more kinds thereof.

The surfactant concentration in the binding solution is, for example, 0.01% (w/v) or more and preferably 0.05% (w/v) or more, and is, for example, 10% (w/v) or less and preferably 3% (w/v) or less. When the surfactant concentration is within the above range, micelles are sufficiently formed, so that the effect of the surfactant can be reliably exhibited.

The first carrier may be any carrier to which the Ng-related peptide can be bound, and examples thereof include an antibody-immobilizing carrier.

The antibody immobilized on the first carrier is an antibody having an antigen binding site capable of recognizing an Ng-related peptide (anti-Ng-related peptide antibody), and examples thereof include an immunoglobulin having an antigen binding site capable of recognizing an Ng-related peptide or a fragment thereof.

Examples of the immunoglobulin include IgG (IgG1, IgG2, IgG3, IgG4), IgM, IgA, IgY, IgD, and IgE. Examples of the immunoglobulin fragment include F(ab')2, F(ab'), F(ab), Fd, Fv, L chain, and H chain. More specifically, clones NG2, NG7, EPR21152, fragments thereof, and the like. The antibody may be either a monoclonal antibody or a polyclonal antibody.

Examples of the material of the first carrier include agarose, sepharose, dextran, silica gel, polyacrylamide, polystyrene, polyethylene, polypropylene, polyester, polyacrylonitrile, a (meth)acrylic acid-based polymer, a fluororesin, a metal complex resin, glass, metal, and a magnetic body.

The shape of the first carrier may be any shape such as a spherical shape (including a bead shape), a plate shape, a needle shape, and an irregular shape, and may be a flow path wall in a microdevice or the like.

Before the first binding step, pretreatment for removing antibodies such as IgG and IgM may be performed as necessary.

### (First washing step)

In the first washing step, after the first binding step, the first conjugate is washed using the first washing solution.

The first washing solution is preferably a neutral buffer containing a surfactant. This makes it possible to effectively remove highly hydrophobic undesirable components (blood proteins, lipids, glycolipids, and the like). Examples of the neutral buffer and the surfactant in the first washing solution include those similar to the neutral buffer and the surfactant exemplified in the binding solution.

The surfactant concentration in the first washing solution is, for example, 0.01% (w/v) or more and preferably 0.02% (w/v) or more, and is, for example, 5% (w/v) or less and preferably 2% (w/v) or less. When the surfactant concentration is within the above range, micelles are sufficiently formed, so that the effect of the surfactant can be reliably exhibited.

As a washing method, a known method may be adopted, and washing is preferably performed a plurality of times. For example, washing is performed using a neutral buffer containing a surfactant, followed by washing using the neutral buffer not containing the surfactant.

As a neutral buffer containing no surfactant, a neutral buffer similar to the neutral buffer exemplified in the binding solution can be used. This makes it possible to suppress foaming caused by the surfactant remaining in the first conjugate.

As the washing method, a general method may be employed, and examples thereof include a method for stirring a carrier in a washing solution, a method for spraying a washing solution from a washing nozzle, and the like. After washing with these neutral buffers, washing with water may be further performed as necessary.

### (First elution step)

In the first elution step, the first conjugate is brought into contact with a first acidic solution after the first washing step. As a result, the Ng-related peptide is dissociated from the first conjugate, and the Ng-related peptide is eluted into the first acidic solution. As a result, a first eluate containing the Ng-related peptide is obtained.

Examples of the first acidic solution include an acidic aqueous solution such as a glycine buffer and hydrochloric acid, and preferably include a glycine buffer. The pH of the first acidic solution is, for example, 3.5 or less and preferably 3.0 or less, and is, for example, 0.5 or more and preferably 1.0 or more.

The first acidic solution preferably contains a surfactant. This makes it possible to more reliably dissociate the Ng-related peptide from the first conjugate. In addition, the eluted Ng-related peptide is inhibited from adhering to a container such as a test tube or a microplate. Therefore, the recovery rate of the Ng-related peptide can be reliably improved, and the detection sensitivity can be improved. Examples of the surfactant used in the first acidic solution include the same surfactants as those exemplified in the binding solution. The surfactant concentration in the first acidic solution is the same as the surfactant concentration in the first washing solution.

### (Neutralization step)

In the neutralization step, after the first elution step, the first eluate is mixed with a neutral buffer. As a result, the first eluate is neutralized to obtain a purified solution containing an Ng-related peptide.

The neutral buffer used in the neutralization step preferably contains a surfactant. This makes it possible to suppress non-specific adsorption to the second conjugate in the second binding step. Examples of the neutral buffer and the surfactant in the neutralization step include those similar to the neutral buffer and the surfactant exemplified in the binding solution. The surfactant concentration in the neutral buffer is the same as the surfactant concentration in the first binding solution.

The pH of the obtained purified solution is neutral, for example, pH 6.0 or more and preferably 6.5 or more, and is, for example, 8.5 or less and preferably 8.0 or less. Accordingly, in the second binding step, the bonding efficiency can be improved.

### (Second binding step)

In the second binding step, the purified solution is brought into contact with a second carrier after the neutralization step. As a result, the Ng-related peptide in the purified solution is bound to the second carrier to obtain a second conjugate.

The second carrier is preferably an antibody-immobilizing carrier, and specific examples thereof include the same antibody-immobilizing carriers as exemplified for the first carrier.

### (Second washing step)

In the second washing step, after the second binding step, the second conjugate is washed using the second washing solution.

The second washing solution is preferably a neutral buffer containing a surfactant. As a result, for example, this makes it possible to effectively remove highly hydrophobic undesirable components (blood proteins, lipids, glycolipids, and the like). Examples of the neutral buffer and the surfactant used in the second washing solution include those similar to the neutral buffer and the surfactant exemplified in the binding solution. The surfactant concentration in the second washing solution is the same as the surfactant concentration in the first washing solution.

As the washing method, a known method may be adopted, and specifically, a method similar to the washing method exemplified in the first washing step may be performed.

### (Second elution step)

In the second elution step, the second conjugate is brought into contact with a second acidic solution (solution for elution) after the second washing step. As a result, the Ng-related peptide is dissociated from the second conjugate, and the Ng-related peptide is eluted into the second acidic solution. As a result, a second eluate containing the Ng-related peptide is obtained.

Examples of the acidic aqueous solution constituting the second acidic solution include those similar to the first acidic solution exemplified in the first elution step, and preferably include hydrochloric acid.

The second acidic solution contains a protein. The mass of the protein is 9 kDa or more, and is, for example, 100 kDa or less, preferably 15 kDa or less, and more preferably 11 kDa or less. By blending the protein having the above mass in the second acidic solution, the protein is contained in the laser irradiation target, and as a result, the detection sensitivity of the Ng-related peptide is improved. In addition, by setting the lower limit of the mass of the protein to 9 kDa, it is possible to avoid overlapping with the mass range of the Ng-related peptide to be analyzed, and it is possible to accurately detect only the Ng-related peptide. By setting the upper limit of the mass of the protein to 100 kDa, preferably 15 kDa, particularly 11 kDa, the detection sensitivity can be remarkably improved.

The protein is not limited as long as it has a mass of 9 kDa or more, and specifically includes recombinant neurogranin, cytochrome, bovine serum albumin (BSA), ovalbumin, lysozyme, and the like.

The concentration of the protein in the second acidic solution, and thus the concentration of the protein in the second eluate (an example of the eluate) is, for example, 10 nM or more, preferably 20 nM or more, and more preferably 30 nM or more, and is, for example, 600 nM or less, preferably 300 nM or less, and more preferably 150 nM or less. In other words, the concentration is, for example, 0.1 pg/mL or more, preferably 0.25 pg/mL or more, and is, for example, 30 pg/mL or less, preferably 4.00 pg/mL or less. By setting the concentration of the protein within the above range, the detection sensitivity can be appropriately improved.

The second acidic solution preferably contains a volatile organic solvent. As a result, the Ng-related peptide can be efficiently dissociated from the second conjugate and eluted in the second acidic solution, and the recovery rate of the Ng-related peptide can be improved.

Examples of the volatile organic solvent include organic solvents miscible with water in an optional ratio, and examples thereof include acetonitrile, methanol, ethanol, acetone, toluene, isopropanol, hexane, butanol, cyclohexane, ethylene glycol, benzene, chloroform, acetaldehyde, triethylamine, phenol, naphthalene, formaldehyde, tetrahydrofuran, and ethyl acetate, and preferred examples thereof include acetonitrile, methanol, ethanol, acetone, and isopropanol. These organic solvents can be used singly or in combination of two or more kinds thereof.

The concentration of the volatile organic solvent in the second acidic solution is, for example, 10% (v/v) or more and preferably 25% (v/v) or more, and is, for example, 90% (v/v) or less and preferably 80% (v/v) or less. When the concentration is within the above range, the Ng-related peptide can be efficiently dissociated from the second carrier, and the sensitivity (S/N ratio) at the time of mass spectrometry can be improved.

The second acidic solution preferably further contains amino acid such as methionine. As a result, the oxidation of the Ng-related peptide can be reduced until the analysis is started after the Ng-related peptide is placed in the mass spectrometer, and the analysis sensitivity can be improved. The amino acid concentration in the second acidic solution is, for example, 0.01 mM or more and preferably 0.05 mM or more, and is, for example, 5 mM or less and preferably 1 mM or less.

### 1-2. Detection step

In the detection step, after the purification step, MALDI-MS (Matrix Assisted Laser Desorption/Ionization-Mass spectrometry) is performed on the second eluate to detect the Ng-related peptide. That is, the Ng-related peptide contained in the second eluate or the dried product thereof is ionized by MALDI, and the ionized peptide is detected by mass spectrometry.

In MALDI-MS, for example, MALDI-TOF (Matrix Assisted Laser Desorption/Ionization-Time of Flight) type mass spectrometer, MALDI-IT (Matrix-Assisted Laser Desorption/Ionization-Ion Trap) type mass spectrometer, MALDI-IT-TOF (Matrix-Assisted Laser Desorption/Ionization-Ion Trap-Time of Flight) type mass spectrometer, MALDI-FTICR (Matrix-Assisted Laser Desorption/Ionization-Fourier Transform Ion Cyclotron Resonance) type mass spectrometer, or the like may be used and operated according to a known method.

In the detection operation of MALDI-MS, first, a laser irradiation target is arranged on a MALDI plate. Specifically, for example, a matrix-containing solution is added dropwise to a MALDI plate and dried (crystallized) to arrange the matrix, and then the second eluate is added dropwise to the matrix and dried. As a result, a laser irradiation target in which the matrix, the Ng-related peptide, and the protein of 9 kD or more exist in a dry state (solid state) is obtained. Note that, if necessary, a laser irradiation target present in a liquid state in which a solid component is concentrated may be obtained without performing complete drying.

Subsequently, the Ng-related peptide is ionized by irradiating the laser irradiation target with a laser, and the ionized peptide is detected by the mass spectrometer. By analyzing the detection result with a mass spectrometer, Ng-related peptides can be quantified.

Examples of the matrix include α-cyano-4-hydroxycinnamic acid (CHCA), 2,5-dihydroxybenzoic acid, sinapinic acid, 3-aminoquinoline, and the like. These matrices can be used singly or in combination of two or more kinds thereof.

Examples of the solvent containing the matrix include acetonitrile, trifluoroacetic acid, methanol, ethanol, and water. These solvents can be used singly or in combination of two or more kinds thereof.

The matrix concentration in the matrix-containing solution is, for example, 0.1 mg/mL or more and preferably 0.5 mg/mL or more, and is, for example, 50 mg/mL or less and preferably 10 mg/mL or less.

The amount of the matrix disposed in the MALDI plate is, for example, 0.1 pg or more and preferably 0.5 pg or more, and is, for example, 50 pg or less and preferably 10 pg or less per well.

Preferably, a matrix additive is used in combination with the matrix. Examples of the matrix additive include a phosphonic acid group-containing compound and an ammonium salt, and preferably include a phosphonic acid group -containing compound from the viewpoint of being able to suppress an adverse effect on the background due to the remaining of the washing solution. Examples of the phosphonic acid group-containing compound include phosphonic acid, methylphosphonic acid, phenylphosphonic acid, 1-naphthylmethylphosphonic acid, methylenediphosphonic acid (MDPNA), ethylenediphosphonic acid, ethane-1-hydroxy-1,1-diphosphonic acid, nitrilotriphosphonic acid, and ethylene diaminotetraphosphonic acid.

The matrix additive concentration in the matrix-containing solution is, for example, 0.01% (w/v) or more and preferably 0.1% (w/v) or more, and is, for example, 10% (w/v) or less and preferably 1% (w/v) or less.

In the laser irradiation target, the content of the protein of 9 kDa or more per 1 pg of the matrix is 10 finol or more and 600 fmol or less. The content is preferably 20 fmol or more and more preferably 30 finol or more, and is preferably 300 finol or less and more preferably 150 finol or less. When the content of the protein is less than the above lower limit, the effect of protein blending does not occur, and the detection sensitivity may not be improved. On the other hand, when the content of the protein exceeds the above upper limit, the detection sensitivity may be lowered as compared with the case where no protein is contained.

Thereby, the Ng-related peptide contained in the second eluate (in turn, the sample) can be measured. In this analysis method, particularly, since the laser irradiation target contains a protein of 9 kDa or more and the content of the protein per 1 pg of the matrix is 10 fmol or more and 600 fmol or less, the Ng-related peptide can be detected with very high sensitivity (S/N). This is because in MALDI-MS, a sample is usually ionized monovalently by receiving protons from a laser-excited matrix, and a peak of the monovalent ion is detected. However, in the Ng-related peptide, due to its structure, divalent ionization also occurs, and the intensity of monovalent ions relatively decreases. On the other hand, in the first embodiment, since a protein of 9 kDa or more coexists in the matrix, the number of proton receptors increases, and protons are dispersed, and therefore divalent ions decrease. Note that the first embodiment is not limited to the above mechanism.

### 2. Second embodiment

In the first embodiment, a protein of 9 kDa or more is added to the second acidic solution in the purification step, but in the second embodiment, in the detection step, the second eluate and a liquid containing a protein of 9 kDa or more (protein-containing solution) are mixed on a plate irradiated with a laser to contain a protein of 9 kDa or more in a laser irradiation target.

The analysis method of the second embodiment sequentially includes a purification step and a detection step. The purification step is, for example, affinity purification, and includes a first binding step, a first washing step, a first elution step, a neutralization step, a second binding step, a second washing step, and a second elution step in this order. The first binding step, the first washing step, the first elution step, the neutralization step, the second binding step, and the second washing step are the same as those steps in the first embodiment. The second elution step in the second embodiment is the same as the second elution step in the first embodiment except that the second acidic solution and thus the second eluate do not contain a protein of 9 kDa or more.

In the detection step, a laser irradiation target is arranged on a MALDI plate. At this time, the protein-containing solution is added dropwise in addition to the matrix-containing solution and the second eluate. Specifically, the matrix-containing solution is added dropwise to the MALDI plate and dried, and subsequently the second eluate and the protein-containing solution are added dropwise thereto and dried. As a result, a laser irradiation target in which the matrix, the Ng-related peptide, and the protein of 9 kD or more are contained in a dry state is obtained. Thereafter, as in the first embodiment, the laser irradiation target is irradiated with a laser, and ionized peptides are detected by a mass spectrometer. Since it is sufficient that the matrix, the Ng-related peptide, and the protein of 9 kD or more are contained in a dry state, the order of dropping of each of the matrix, the Ng-related peptide, and the protein of 9 kD or more is not limited.

The protein-containing solution contains a protein of 9 kDa or more and a solvent. Examples of the solvent include, but are not limited to, a solvent similar to the solvent containing the matrix, preferably a solvent used for the second acidic solution. The protein concentration in the protein-containing solution and the dropping amount thereof may be adjusted so that the protein concentration per 1 pg of the matrix is 10 finol or more and 600 fmol or less (preferably 20 fmol or more, more preferably 30 finol or more, and preferably 300 finol or less and 150 finol or less). Specifically, the concentration of the protein may be, for example, 10 nM or more and preferably 50 nM or more, and may be, for example, 1000 nM or less and preferably 600 nM or less, and the dropping amount may be, for example, 0.1 pL or more and preferably 0.2 pL or more, and may be, for example, 10 pL or less and preferably 5 pL or less.

The second embodiment also has the same effects as those of the first embodiment. The first embodiment is preferable from the viewpoint that the number of accurate dropping operation steps onto the MALDI plate does not increase and the laser irradiation target is easily mixed uniformly.

### 3. Third and fourth embodiments

In the analysis methods of the first and second embodiments, immunoprecipitation is performed a plurality of times (twice) as the purification step, but for example, immunoprecipitation may be performed only once.

Specifically, the analysis method of the third embodiment includes a first binding step (binding step), a first washing step (washing step), a second elution step (elution step), and a detection step, and in the second elution step, the second acidic solution (solution for elution) contains a protein of 9 kDa or more.

The analysis method of the fourth embodiment includes a first binding step (binding step), a first washing step (washing step), a second elution step (elution step), and a detection step, and in the detection step, the second eluate and the protein-containing solution are mixed on a plate.

The third and fourth embodiments also have the same effects as those of the first embodiment. From the viewpoint that the Ng-related peptide can be reliably analyzed even when the amount of the Ng-related peptide present in the sample is even smaller, the first and second embodiments are preferred.

### 4. Aspects

It is understood by those skilled in the art that the plurality of exemplary embodiments described above are specific examples of the following aspects.

(Aspect 1) An analysis method according to one aspect is a method for analyzing a neurogranin-related peptide by matrix assisted laser desorption/ionization-mass spectrometry, wherein a laser irradiation target may contain the neurogranin-related peptide, a matrix, and a protein of 9 kDa or more, and a content of the protein per 1 pg of the matrix may be 10 finol or more and 600 fmol or less.

(Aspect 2) In the method according to Aspect 1, the content of the protein per 1 pg of the matrix may be 30 fmol or more and 150 fmol or less.

(Aspect 3) In the method according to item 1 or 2, a mass of the protein may be 9 kDa or more and 15 kDa or less.

(Aspect 4) The method according to any one of Aspects 1 to 3, sequentially includes: a binding step of bringing a sample containing a neurogranin-related peptide into contact with a carrier in a binding solution to obtain a conjugate in which the neurogranin-related peptide is bound to the carrier; a washing step of washing the conjugate using a washing solution; an elution step of bringing the conjugate into contact with an acidic solution to obtain an eluate in which the neurogranin-related peptide is eluted in the acidic solution; and a detection step of performing matrix assisted laser desorption/ionization-mass spectrometry on the eluate to detect the neurogranin-related peptide, wherein the acidic solution may contain the protein.

(Aspect 5) In the method according to Aspect 4, a concentration of the protein in the eluate may be 10 nM or more and 600 nM or less.

(Aspect 6) The method according to any one of Aspects 1 to 3, sequentially includes: a binding step of bringing a sample containing a neurogranin-related peptide into contact with a carrier in a binding solution to obtain a conjugate in which the neurogranin-related peptide is bound to the carrier; a washing step of washing the conjugate using a washing solution; an elution step of bringing the conjugate into contact with an acidic solution to obtain an eluate in which the neurogranin-related peptide is eluted in the acidic solution; and a detection step of performing matrix assisted laser desorption/ionization-mass spectrometry on the eluate to detect the neurogranin-related peptide, wherein in the detection step, the eluate and a liquid containing the protein may be mixed on a plate irradiated with a laser.

(Aspect 7) In the method according to Aspect 6, a concentration of the protein in the liquid containing the protein may be 10 nM or more and 600 nM or less.

### EXAMPLES

Next, the present invention will be described in detail with reference to Examples, but the scope of the present invention is not limited thereto.

### <Example 1>

### (First binding step, first washing step, and first elution step)

A clone NG2 (BioLegend, Inc.) of an anti-Ng antibody (IgG1) having 52-63 residues of human Neurogranin (Ng) as an epitope was prepared. To 100 pg of an anti-Ng antibody, 5.5 mg of magnetic beads (Dynabeads M-270 Epoxy) were added in an immobilization buffer (0.1 M phosphate buffer containing 1.5 M ammonium sulfate; pH 7.4) at 37°C for 16 to 24 hours. Thus, antibody beads were produced.

250 µL of a sample containing 2 kinds of Ng peptides (each peptide concentration: 40 pM) described in the following Table 1 was prepared. The sample was mixed with 250 pL of a binding buffer (0.1% n-undecyl-β-D-maltoside (UDM), 800 mM GlcNAc, 100 mM Tris-HCl, 300 mM NaCl; pH 7.4) containing 20 pM of stable isotope labeled Ng 50-78 (SIL-Ng50-78) and allowed to stand on ice for 5 to 60 minutes. The mixed sample was mixed with the antibody beads and shaken on ice for 1 hour. Thereafter, the antibody beads were washed 3 times with 100 pL of the first washing buffer (0.05% UDM, 50 mM Tris-HCl, 150 mM NaCl; pH 7.4) and 2 times with 50 µL of 50 mM ammonium acetate buffer. Thereafter, the antibody beads were brought into contact with the first acidic solution (0.05% UDM, 50 mM glycine buffer; pH 2.8) to elute the Ng-related peptide into the first acidic solution. Thus, a first eluate containing the Ng-related peptide was obtained.

**[Table 1]**

| Peptide name | Sequence | SEQ ID NO. | Mass |
|---|---|---|---|
| Ng43-75 | RKKIKSGERGRKGPGPGGPGGAGVARGGAGGGP | 1 | 2984.380a |
| Ng33-75 | IQASFRGHMARKKIKSGERGRKGPGPGGPGGAGVARGGAGGGP | 2 | 4083.650a |

### (Neutralization step)

The first eluate was mixed with a neutral buffer (0.1% UDM, 800 mM GlcNAc, 300 mM Tris-HCl, 300 mM NaCl; pH 7.4) to give a purified solution.

### (Second binding step, second washing step, and second elution step)

The purified solution was mixed with antibody beads and shaken on ice for 1 hour. Thereafter, the antibody beads were washed 5 times with 50 µL of the second washing buffer (0.05% UDM, 50 mM Tris-HCl, 150 mM NaCl; pH 7.4) and 2 times with 50 µL of 50 mM ammonium acetate buffer, and washed once with 30 µL of water. Thereafter, the antibody beads were brought into contact with 5 µL of a second acidic solution (70% (v/v) acetonitrile aqueous solution with amounts shown in Table 2 of recombinant Ng, 5 mM hydrochloric acid and 0.1 mM methionine) to elute the Ng-related peptide into the second acidic solution. Thus, a second eluate containing the Ng-related peptide was obtained.

### (Detection step)

AXIMA Performance (Shimadzu/KRATOS, Manchester, UK), which is a MALDI-TOF MS apparatus, was used as a mass spectrometer. Using α-cyano-4-hydroxycinnamic acid (CHCA) as a matrix for Linear TOF, methylene diphosphonic acid (MDPNA) as a matrix additive and acetonitrile as a solvent, a 2 mg/mL CHCA/0.2% (w/v) MDPNA matrix solution was prepared. 0.5 µL each (that is, the matrix is 1 µg per well) of the matrix solution was added dropwise to 4 wells of a MALDI plate (µFocus MALDI plate 900 µm (Hudson Surface Technology, Inc., Fort Lee, NJ)) and dried, and then 1 µL each of the second eluate was further added dropwise thereto and dried.

Then, MALDI-TOF MS was then activated to detect Ng-related peptides (Ng43-75 and Ng33-75). As a setting condition, mass spectrum data was acquired by Linear TOF in a positive ion mode. 400 spots and 16000 shots were accumulated for 1 well. A m/z value of Linear TOF was expressed as a peak average mass. The m/z value was calibrated using human angiotensin II and human ACTH fragment 18-39, bovine insulin oxidized beta-chain, bovine insulin, and cytochrome c as external standards.

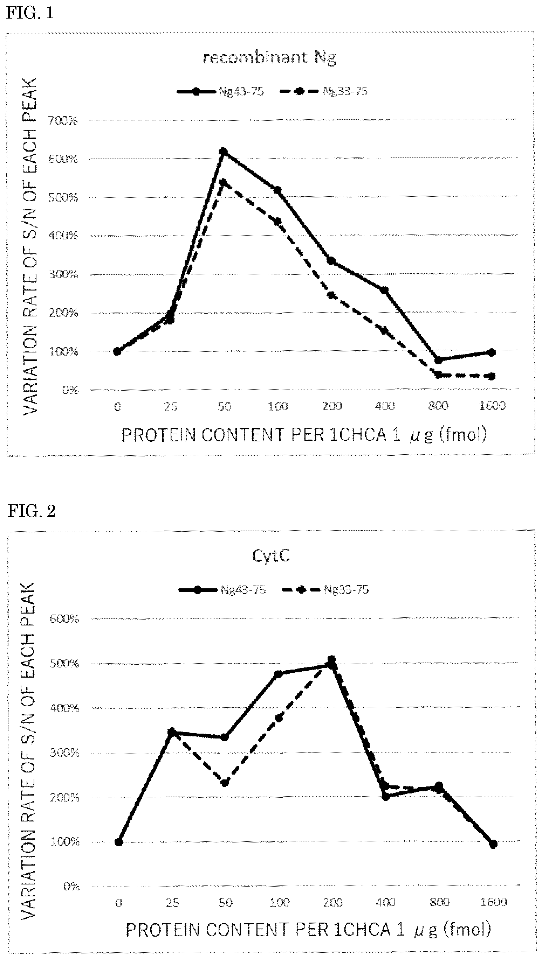

At this time, the concentration of the protein (recombiant Ng) contained in the second acidic solution and thus the second eluate was set to a plurality of concentrations (0 to 1600 nM) shown in the following Table 2, and the measurement was performed. The signal/noise ratio (S/N) of the peak in the case of containing the amount of protein in Table 1 (concentration of 25 to 1600 nM) to the S/N of the peak in the case of containing no protein (concentration of 0 nM) was calculated, and these graphs are shown in FIG. 1.

**[Table 2]**

| Example 1 | | | | Example 2 | | | | Example 3 | | | |
|---|---|---|---|---|---|---|---|---|---|---|---|
| Protein : recombinant Ng | | | | Protein : CytC | | | | Protein : BSA | | | |
| Concentration in second acidic solution | | Content per CHCA 1 *µ* g | | Concentration in second acidic solution | | Content per CHCA 1 *µ* g | | Concentration in second acidic solution | | Content per CHCA 1 *µ* g | |
| *µ* g/mL | nM | ng | fmol | *µ* g/mL | nM | ng | fmol | *µ* g/mL | nM | ng | fmol |
| 0 | 0 | 0 | 0 | 0 | 0 | 0 | 0 | 0 | 0 | 0 | 0 |
| 0.25 | 25 | 0.25 | 25 | 0.31 | 25 | 0.31 | 25 | 1.66 | 25 | 1.66 | 25 |
| 0.5 | 50 | 0.5 | 50 | 0.62 | 50 | 0.62 | 50 | 3.31 | 50 | 3.31 | 50 |
| 0.99 | 100 | 0.99 | 100 | 1.24 | 100 | 1.24 | 100 | 6.63 | 100 | 6.63 | 100 |
| 1.99 | 200 | 1.99 | 200 | 2.47 | 200 | 2.47 | 200 | 13.26 | 200 | 13.26 | 200 |
| 3.97 | 400 | 3.97 | 400 | 4.94 | 400 | 4.94 | 400 | 26.52 | 400 | 26.52 | 400 |
| 7.94 | 800 | 7.94 | 800 | 9.89 | 800 | 9.89 | 800 | 53.04 | 800 | 53.04 | 800 |
| 15.88 | 1600 | 15.88 | 1600 | 19.78 | 1600 | 19.78 | 1600 | 106.07 | 1600 | 106.07 | 1600 |

### <Examples 2 and 3>

The analysis method was performed in the same manner as in Example 1 except that the type and concentration of the protein contained in the second acidic solution were changed to the type and concentration shown in Table 2. These results are shown in FIGS. 2 to 3. From FIGS. 1 to 3, it can be seen that the detection sensitivity is improved when the content of the protein per 1 pg of the matrix is 10 to 600 fmol.

In the proteins used, the mass of recombinant Ng (rNg; Abeam corporation) was 9925.92 Da, the mass of Cytochrome C (CytC; SIGMA corporation) was 12360.52 Da, and the mass of Bovine sewrum albumin (BSA; manufactured by nacalai tesque corporation) was 66296 Da.

### <Example 4>

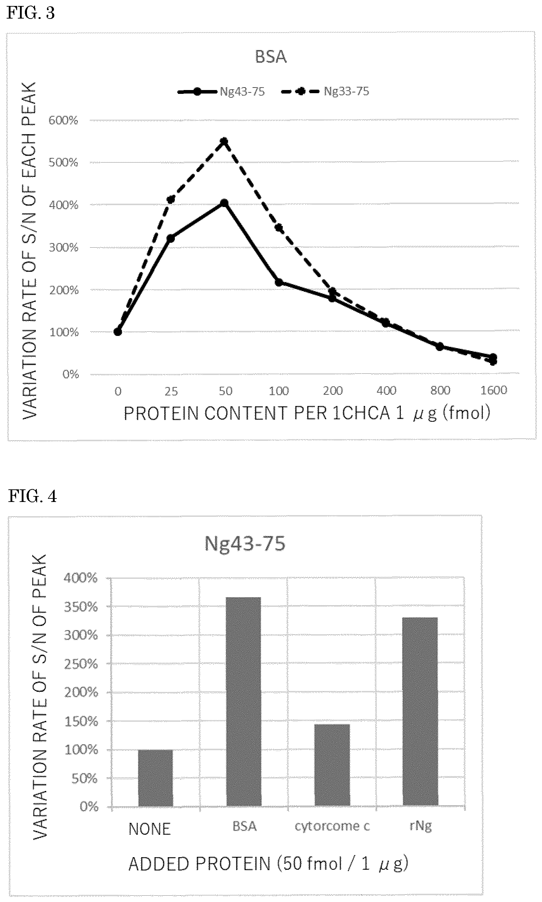

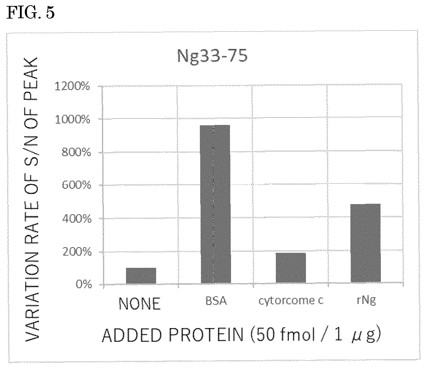

This example was performed in the same manner as in Examples 1 to 3. However, instead of 5 pL of the second acidic solution containing protein (rNg, CytC, BSA), 2.5 pL of the second acidic solution not containing protein was used. Further, 0.5 pL of the matrix solution was added dropwise to each well of the MALDI plate and dried, and then 0.5 pL of the second eluate and 0.5 pL of a protein-containing solution (protein concentration 100 nM, solvent: 70% (v/v) acetonitrile aqueous solution containing 5 mM hydrochloric acid and 0.1 mM methionine) were added dropwise thereto and dried. The blending amount of the protein per 1 µg of the matrix at this time was 50 fmol. For the Ng43-75 peptide, the signal/noise ratio (S/N) results of the peak in the case of containing protein to the S/N of the peak in the case of not containing protein (no added protein) for each protein (rNg, CytC, BSA) are shown in FIG. 4. For the Ng33-75 peptide, the signal/noise ratio (S/N) results of the peak in the case of containing protein to the S/N of the peak in the case of not containing protein (no added protein) for each protein (rNg, CytC, BSA) are shown in FIG. 5.

## Claims

1. A method for analyzing a neurogranin-related peptide by matrix assisted laser desorption/ionization-mass spectrometry,
wherein a laser irradiation target contains the neurogranin-related peptide, a matrix, and a protein of 9 kDa or more, and
a content of the protein per 1 pg of the matrix is 10 finol or more and 600 fmol or less.

2. The method according to claim 1, wherein the content of the protein per 1 pg of the matrix is 30 fmol or more and 150 fmol or less.

3. The method according to claim 1, wherein a mass of the protein is 9 kDa or more and 15 kDa or less.

4. The method according to claim 1, sequentially comprising:
a binding step of bringing a sample containing a neurogranin-related peptide into contact with a carrier in a binding solution to obtain a conjugate in which the neurogranin-related peptide is bound to the carrier;
a washing step of washing the conjugate using a washing solution;
an elution step of bringing the conjugate into contact with an acidic solution to obtain an eluate in which the neurogranin-related peptide is eluted in the acidic solution; and
a detection step of performing matrix assisted laser desorption/ionization-mass spectrometry on the eluate to detect the neurogranin-related peptide,
wherein the acidic solution contains the protein.

5. The method according to claim 4, wherein a concentration of the protein in the eluate is 10 nM or more and 600 nM or less.

6. The method according to claim 1, sequentially comprising:
a binding step of bringing a sample containing a neurogranin-related peptide into contact with a carrier in a binding solution to obtain a conjugate in which the neurogranin-related peptide is bound to the carrier;
a washing step of washing the conjugate using a washing solution;
an elution step of bringing the conjugate into contact with an acidic solution to obtain an eluate in which the neurogranin-related peptide is eluted in the acidic solution; and
a detection step of performing matrix assisted laser desorption/ionization-mass spectrometry on the eluate to detect the neurogranin-related peptide,
wherein in the detection step, the eluate and a liquid containing the protein are mixed on a plate irradiated with a laser.

7. The method according to claim 6, wherein a concentration of the protein in the liquid containing the protein is 10 nM or more and 600 nM or less.
